# EUROPEAN PATENT APPLICATION

(11) **EP 4 303 804 A1**
(43) Date of publication of application: **10.01.2024**
(21) Application number: 22763277.5
(22) Date of filing: 01.03.2022
(51) Int. Cl.: G06Q 40/08, G16H 50/30

(54) **DISEASE PREDICTING SYSTEM, INSURANCE PREMIUM CALCULATING SYSTEM, AND DISEASE PREDICTING METHOD**

(30) Priority: 04.03.2021 JP 2021034812
(71) Applicant: Anicom Holdings, Inc., Shinjuku-ku Tokyo 160-0023 (JP)
(72) Inventor: KOIZUMI Akihito, Tokyo 160-0023 (JP)
(74) Representative: Gill Jennings & Every LLP
(86) International application number: PCT/JP2022/008666
(87) International publication number: WO 2022/186223

(57) **Abstract**

An object is to provide a disease predicting system, a disease predicting method, and an insurance premium calculating system, for animals. The disease predicting system includes receiving means of receiving occupancy rate data and diversity data of intestinal flora of an animal other than humans and determining means of predicting and determining, using a learned model, whether the animal will be afflicted with a disease from the occupancy rate data and diversity data of the intestinal flora of the animal input to the receiving means.

## Description

### TECHNICAL FIELD

The present invention relates to a disease predicting system, an insurance premium calculating system, and a disease predicting method, and more particularly to a disease predicting system, an insurance premium calculating system, and a disease predicting method that provides information on the likelihood of future disease affliction in animals from data on intestinal flora of the animals.

### BACKGROUND ART

Pet animals such as dogs, cats, and rabbits and domestic animals such as cattle and pigs are irreplaceable to humans. In recent years, while the average lifespan of animals raised by humans has increased significantly, such animals are more likely to be afflicted with some kind of disease during their lifetime, and increasing medical costs borne by their owners have become a problem.

In order to maintain the health of animals, it is important to manage their physical condition through daily diet, exercise, and the like and to quickly respond to their physical problem. However, animals are unable to express their poor physical problems in their own language, due to which in fact, animal owners do not become aware that their animals are afflicted with a disease until symptoms progress and some externally observable signs occur.

Therefore, there is a need for a simple means to know whether an animal is likely to be afflicted with a disease in the future. Particularly, it is useful to be able to know the likelihood of disease affliction in the future when an animal is not afflicted with a disease or has no symptoms because specific steps can be taken to prevent the disease.

Patent Document 1 discloses an intestinal flora regulating or improving composition that has an effect of effectively regulating or improving intestinal flora by increasing bacteria belonging to the phylum Bacteroidetes and decreasing bacteria belonging to the phylum Firmicutes in the intestinal flora, but does not disclose any method for predicting whether an animal will be afflicted with a disease from data on intestinal flora of the animal.

### RELATED ART DOCUMENT

### PATENT DOCUMENT

[Patent Document 1] WO 2017/094892

### SUMMARY OF THE INVENTION

### PROBLEMS TO BE SOLVED BY THE INVENTION

Accordingly, it is an object of the present invention to provide a disease predicting system and the like that predict the likelihood that an animal will be afflicted with a disease in the future using a simple method.

### MEANS FOR SOLVING THE PROBLEMS

The present inventor analyzed and examined data on intestinal flora of animals covered by pet insurance and a large amount of data on the presence or absence of insurance claims for the animals, i.e., the presence or absence of disease affliction, and, as a result, has found that it is possible to predict whether the animals will be affected with disease in the future using the data on the intestinal flora of the animals, thereby having completed the present invention.

Specifically, the present invention is the following [1] to [11].
[1] A disease predicting system including receiving means of receiving occupancy rate data and diversity data of intestinal flora of an animal other than humans and determining means of predicting and determining, using a learned model, whether the animal will be afflicted with a disease from the occupancy rate data and diversity data of the intestinal flora of the animal input to the receiving means.
[2] The disease predicting system according to [1], in which the learned model performs learning using, as teacher data, the occupancy rate data and diversity data of the intestinal flora of the animal other than humans and a presence or absence of disease affliction within a predetermined period of time from a time of acquisition of the occupancy rate data and diversity data of the intestinal flora of the animal, and in which an input is occupancy rate data and diversity data of intestinal flora of an animal and an output is a prediction determination of whether the animal will be afflicted with a disease.
[3] The disease predicting system according to [2], in which the occupancy rate data includes an occupancy rate of each family or a label set based on the occupancy rate of each family.
[4] The disease predicting system according to [3], in which the occupancy rate data includes an occupancy rate of one or more families selected from the group consisting of Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, and Veillonellaceae or a label set based on the occupancy rate.
[5] The disease predicting system according to [4], in which the occupancy rate data further includes an occupancy rate of Streptococcaceae or a label set based on the occupancy rate.
[6] The disease predicting system according to [4], in which the occupancy rate data further includes an occupancy rate of one or more families selected from the group consisting of Campylobacteraceae, Desulfovibrionaceae, Flavobacteriaceae, Helicobacteraceae, Odoribacteraceae, Paraprevotellaceae, Peptococcaceae, Porphyromonadaceae, and Succinivibrionaceae or a label set based on the occupancy rate.
[7] The disease predicting system according to any of [1] to [6], in which the diversity data includes a diversity index or a label set based on the diversity index.
[8] An insurance premium calculating system that inputs occupancy rate data and diversity data of intestinal flora of an animal covered by insurance to the disease predicting system according to any of [1] to [7] and determines an insurance premium for the animal according to the output prediction of disease affliction.
[9] A learned model generating method for generating a learned model that predicts from occupancy rate data and diversity data of intestinal flora of an animal other than humans whether the animal will be afflicted with a disease within a predetermined period of time, the method including: inputting as teacher data occupancy rate data and diversity data of intestinal flora of an animal other than humans and a presence or absence of disease affliction within a predetermined period of time from a time of acquisition of the occupancy rate data and diversity data of the intestinal flora of the animal to a computer including artificial intelligence; and making artificial intelligence learn.
[10] A disease predicting method including: a step of acquiring occupancy rate data and diversity data of intestinal flora of an animal other than humans; and a step of inputting the occupancy rate data and diversity data to a learned model and causing a computer to output, using the learned model, a prediction of whether the animal will be afflicted with a disease within a predetermined period of time from the input occupancy rate data and diversity data.
[11] The disease predicting method according to [10], in which the learned model performs learning using, as teacher data, the occupancy rate data and diversity data of the intestinal flora of the animal other than humans and a presence or absence of disease affliction within a predetermined period of time from a time of acquisition of the occupancy rate data and diversity data of the intestinal flora of the animal, and in which an input is occupancy rate data and diversity data of intestinal flora of an animal and an output is a prediction determination of whether the animal will be afflicted with a disease.

### EFFECTS OF THE INVENTION

The present invention can provide the disease predicting system, the insurance premium calculating system, the learned model generating method, and the disease predicting method that predict the likelihood that an animal will be afflicted with a disease in the future.

### BRIEF DESCRIPTION OF THE DRAWINGS

FIG. 1 is a schematic diagram of an insurance premium calculating system.
FIG. 2 is a graph chart showing results of Example 1, which is a graph chart showing features that were important for predicting cardiovascular disease affliction in dogs.
FIG. 3 is a graph chart showing results of Example 1, which is a graph chart showing features that were important for predicting respiratory disease affliction in dogs.
FIG. 4 is a graph chart showing results of Example 1, which is a graph chart showing features that were important for predicting gastrointestinal disease affliction in dogs.
FIG. 5 is a graph chart showing results of Example 1, which is a graph chart showing features that were important for predicting hepatic, biliary tract, and pancreatic disease affliction in dogs.
FIG. 6 is a graph chart showing results of Example, which is a graph chart showing features that were important for predicting urologic disease affliction in dogs.
FIG. 7 is a graph chart showing results of Example 1, which is a graph chart showing features that were important for predicting genital disease affliction in dogs.
FIG. 8 is a graph chart showing results of Example 1, which is a graph chart showing features that were important for predicting neurological disease affliction in dogs.
FIG. 9 is a graph chart showing results of Example 1, which is a graph chart showing features that were important for predicting eye and appendage disease affliction in dogs.
FIG. 10 is a graph chart showing results of Example 1, which is a graph chart showing features that were important for predicting ear disease affliction in dogs.
FIG. 11 is a graph chart showing results of Example 1, which is a graph chart showing features that were important for predicting dental and oral disease affliction in dogs.
FIG. 12 is a graph chart showing results of Example 1, which is a graph chart showing features that were important for predicting musculoskeletal disease affliction in dogs.
FIG. 13 is a graph chart showing results of Example 1, which is a graph chart showing features that were important for predicting skin disease affliction in dogs.
FIG. 14 is a graph chart showing results of Example 1, which is a graph chart showing features that were important for predicting blood and hematopoietic disease affliction in dogs.
FIG. 15 is a graph chart showing results of Example 1, which is a graph chart showing features that were important for predicting endocrine disease affliction in dogs.
FIG. 16 is a graph chart showing results of Example 1, which is a graph chart showing features that were important for predicting systemic disease affliction in dogs.
FIG. 17 is a graph chart showing results of Example 2, which is a graph chart showing features that were important for predicting cardiovascular disease affliction in cats.
FIG. 18 is a graph chart showing results of Example 2, which is a graph chart showing features that were important for predicting respiratory disease affliction in cats.
FIG. 19 is a graph chart showing results of Example 2, which is a graph chart showing features that were important for predicting gastrointestinal disease affliction in cats.
FIG. 20 is a graph chart showing results of Example 2, which is a graph chart showing features that were important for predicting hepatic, biliary tract, and pancreatic disease affliction in cats.
FIG. 21 is a graph chart showing results of Example 2, which is a graph chart showing features that were important for predicting urologic disease affliction in cats.
FIG. 22 is a graph chart showing results of Example 2, which is a graph chart showing features that were important for predicting genital disease affliction in cats.
FIG. 23 is a graph chart showing results of Example 2, which is a graph chart showing features that were important for predicting neurological disease affliction in cats.
FIG. 24 is a graph chart showing results of Example 2, which is a graph chart showing features that were important for predicting eye and appendage disease affliction in cats.
FIG. 25 is a graph chart showing results of Example 2, which is a graph chart showing features that were important for predicting ear disease affliction in cats.
FIG. 26 is a graph chart showing results of Example 2, which is a graph chart showing features that were important for predicting dental and oral disease affliction in cats.
FIG. 27 is a graph chart showing results of Example 2, which is a graph chart showing features that were important for predicting musculoskeletal disease affliction in cats.
FIG. 28 is a graph chart showing results of Example 2, which is a graph chart showing features that were important for predicting skin disease affliction in cats.
FIG. 29 is a graph chart showing results of Example 2, which is a graph chart showing features that were important for predicting blood and hematopoietic disease affliction in cats.
FIG. 30 is a graph chart showing results of Example 2, which is a graph chart showing features that were important for predicting endocrine disease affliction in cats.
FIG. 31 is a graph chart showing results of Example 2, which is a graph chart showing features that were important for predicting systemic disease affliction in cats.

### MODE FOR CARRYING OUT THE INVENTION

### [Disease Predicting System]

A disease predicting system of the present invention includes receiving means of receiving input of occupancy rate data and diversity data of intestinal flora of an animal other than humans and determining means using a learned model, determining and outputting a prediction of disease affliction of the animal from the occupancy rate data and diversity data of the intestinal flora of the animal input to the receiving means.

### [Receiving Means]

The receiving means of the present invention is a means of receiving input of occupancy rate data and diversity data of intestinal flora of an animal for which a prediction of disease affliction is desired. As the animal, there are mentioned dogs, cats, birds, rabbits, ferrets, and the like. A method of receiving the occupancy rate data and diversity data may be any method, such as input or transmission of the data to a terminal.

### [Occupancy Rate Data]

Occupancy rate data is data related to the occupancy rate of each bacterium contained in the intestinal flora of an animal. Occupancy rate is an abundance ratio (detection ratio) of each bacterial species in the intestinal flora, and can be measured, for example, as a detection result "hit rate" in a known metagenome analysis method, such as amplicon sequencing using a sequencer such as NGS. The present invention can use an occupancy rate of intestinal flora or a label set based on the occupancy rate, as occupancy rate data.

The occupancy rate may be an occupancy rate at any level of phylum, class, order, family, genus, or species of bacteria, but an occupancy rate of each family of bacteria is preferable. The occupancy rate of each family refers to an occupancy rate of all bacteria belonging to a given family. In other words, when calculating the occupancy rate of each family, for each bacterial species in the intestinal flora, occupancy rates of bacterial species belonging to a given family are totalized to calculate an occupancy rate of the family. Identification to species level or genus level may be performed to totalize for each family, or identification at family level may be performed to calculate an occupancy rate of the family without identifying to species level or genus level.

The label set on the basis of the occupancy rate is a label appropriately set according to the magnitude of a numerical value of the occupancy rate. For example, three levels of labels: "large", "medium", and "small" or "many", "medium", and "few" can be set according to the numerical value of the occupancy rate. Additionally, the number of levels of labels can be optionally set, and, for example, multi-levels of labels, such as "0", "1", "2", "3", ..., "20" can also be attached.

When using a label, an occupancy rate in the intestinal flora is measured, and before inputting a numerical value thereof to the input means, a specific label is assigned from a predetermined correspondence table according to the measured occupancy rate, and the label can be input to the receiving means.

### [Diversity Data]

Diversity data is data related to the diversity of bacteria in the intestinal flora of an animal. Great diversity of the intestinal flora means that the intestinal flora contains a wide variety of different types of bacteria. An index that indicates diversity, a so-called diversity index has several types, and any known index may be used in the present invention. As the diversity index, for example, there are mentioned Shannon-Wiener's diversity index and Simpson's Diversity Index.

### [Measurement of Occupancy Rate Data and Diversity Data]

Occupancy rate data and diversity data of intestinal flora can be measured using a known metagenomic analysis method or bacterial flora analysis method, such as amplicon sequencing using a sequencer such as NGS. For example, there is mentioned a method in which a sample such as feces is collected from an animal, and DNA and RNA base sequence information of any organisms contained in the sample is analyzed using a next-generation sequencer to identify the organisms contained in the sample. Preferably, there is mentioned a method in which all or part of a 16S rRNA gene contained in the sample is amplified as necessary to be sequenced, and the obtained sequence is analyzed using software to obtain composition data of bacteria in the sample. By processing the composition data of the bacteria in the sample with software or by referring to genetic databases such as Genbank, Greengenes, and SILVA database, attribution of bacterial species of the bacteria contained in the sample can be determined, and the occupancy rate data and diversity data of the intestinal flora of the animal can be measured.

An example of amplicon analysis (bacterial flora analysis) of the 16S rRNA gene using NGS (next-generation sequencer) is specifically described. First, DNA is extracted from a sample such as feces using a DNA extraction reagent, and a 16S rRNA gene is amplified from the extracted DNA by PCR. After that, the amplified DNA fragments are comprehensively sequenced using NGS, and after removing low-quality reads and chimeric sequences, the sequences are clustered, and operational taxonomic unit (OTU) analysis is performed. The OTU is an operational classification unit for treating sequences having a certain degree or more of similarity (e.g., 96 to 97% or more of homology) to each other as if they were a single bacterial species. Accordingly, it is considered that the number of OTUs represents the number of bacterial species that make up the bacterial flora, and the number of reads belonging to the same OTU represents a relative abundance of the species. It is also possible to select a representative sequence from among the number of reads belonging to each OTU and to identify a family name and genus and species names by database search. In this way, the occupancy rate of bacteria belonging to a specific family and the diversity index of the intestinal flora can be measured.

### [Determining Means]

The determining means of the present invention includes a learned model which performs learning using, as teacher data, occupancy rate data and diversity data of intestinal flora of an animal other than humans and a fact of disease affliction within a predetermined period of time from a time of acquisition of the occupancy rate data and diversity data of the intestinal flora of the animal, and in which an input is occupancy rate data and diversity data of intestinal flora of an animal, and an output is a prediction that the animal will be afflicted with a disease. Regarding the time of acquisition of the occupancy rate data and diversity data of the intestinal flora, a time of acquisition of a sample to be measured to obtain occupancy rate data and diversity data by measurement may be the time of acquisition of the occupancy rate data and diversity data of the intestinal flora. In other words, in the present specification, when "the time of acquisition of the occupancy rate data and diversity data of the intestinal flora" is referred to, it may include either the time of acquisition of the occupancy rate data and diversity data of the intestinal flora or the time of acquisition of the sample to be measured to obtain occupancy rate data and diversity data by measurement.

As the learned model, artificial intelligence (AI) is preferable. Artificial intelligence (AI) is software or a system that allows a computer to imitate intellectual work performed by human brain, and specifically refers to a computer program or the like that understands natural language used by humans, make logical inferences, and learns from experience. Artificial intelligence may be either a general-purpose type or a specialized type, may be any of a deep neural network, a convolutional neural network, or the like, and published software can be used.

In order to generate a learned model, artificial intelligence is made to learn using teacher data. The learning may be either machine learning or deep learning (deep learning), but machine learning is preferable. Deep learning is advanced machine learning, and is characterized by automatically finding features. The present invention uses occupancy rate data and diversity data of intestinal flora as features.

A learning method for generating a learned model is not particularly limited, and published software can be used. For example, the Deep Learning GPU Training System (DIGITS) published by NVIDIA can be used. In addition, learning may be performed by, for example, a known support vector machine method (Support Vector Machine method) or the like published in "An Introduction to Support Vector Machine" (Kyoritsu Shuppan) and the like.

Machine learning can be either unsupervised learning or supervised learning, but supervised learning is preferable. The method for supervised learning is not particularly limited, and for example, there are mentioned decision tree (decision tree), ensemble learning, gradient boosting, and the like. As published machine learning algorithms, for example, XGBoost, CatBoost, and LightGBM are mentioned.

The teacher data for learning is occupancy rate data and diversity data of the intestinal flora of an animal and the presence or absence of disease affliction, i.e., whether or not the animal has been afflicted with a disease within a predetermined period of time, preferably within 3 years, more preferably within 2 years, still more preferably 1 year, and particularly preferably within 180 days, from a time of acquisition of the occupancy rate data and diversity data of the intestinal flora. Whether or not the animal has been afflicted with a disease can be replaced by a dummy variable. The occupancy rate data and diversity data of the intestinal flora of the animal as teacher data are the same as the occupancy rate data and diversity data of the intestinal flora described in the receiving method above. Information on whether or not the animal has been afflicted with a disease can be obtained, for example, from a veterinary hospital, an insured owner, or the like, as an insurance claim fact (also referred to as an "accident"). In other words, when the animal is an animal covered by pet insurance, and if the animal is diagnosed with a disease at a hospital, the veterinary hospital or the owner (a pet insurance policy holder) will make a claim to the insurance company for payment along with the fact of disease affliction, so that the insurance company can know that the animal has been afflicted with the disease. On the other hand, if no insurance claim is filed until a predetermined time has elapsed from the time of acquisition of the occupancy rate data and diversity data of the intestinal flora, it can be determined that the insured animal was not afflicted with a disease during the period of time.

The learned model may be generated for each individual disease, or may be generated collectively for a plurality of diseases. When generating a learned model for each individual disease, learning is performed using, as teacher data, occupancy rate data and diversity data of intestinal flora of animals afflicted with a specific disease acquired before a predetermined period of time since the disease affliction, the fact of affliction with the disease, occupancy rate data and diversity data of intestinal flora of animals as comparison subjects that were not afflicted with the disease for a predetermined period of time from the time of acquisition of the occupancy rate data and diversity data of the intestinal flora, and the fact of no affliction with the disease for the predetermined period of time. When learning for a plurality of diseases collectively, a plurality of types of teacher data may be prepared as teacher data, such as animals afflicted with a certain disease and occupancy rate data and diversity data of intestinal flora acquired before a predetermined period of time has elapsed since the disease affliction, animals afflicted with another disease other than the disease and occupancy rate data and diversity data of intestinal flora acquired before a predetermined period of time since the disease affliction, and animals afflicted with yet another disease and occupancy rate data and diversity data of intestinal flora acquired before a predetermined period of time has elapsed since the disease affliction.

### [Diseases]

The types of diseases targeted in the present invention are not particularly limited, and, for example, there are mentioned skin diseases, otological diseases, musculoskeletal diseases, ophthalmic diseases, gastrointestinal diseases, systemic diseases, urological diseases, hepatic, biliary tract, and pancreatic diseases, cardiovascular diseases, neurological diseases, respiratory diseases, dental and oral diseases, endocrine diseases, genital system diseases, and blood and hematopoietic diseases.

As skin diseases, for example, dermatitis, atopic dermatitis, and pyoderma are mentioned.

As otologic diseases, for example, otitis externa and otitis media are mentioned.

As musculoskeletal diseases, for example, patellar luxation and intervertebral disc herniation are mentioned.

As ophthalmic diseases, for example, there are mentioned conjunctivitis, eye mucus, keratitis, corneal ulcers/erosions, lacrimation, cataracts, and glaucoma.

As gastrointestinal diseases, for example, gastritis and enteritis are mentioned.

As systemic diseases, for example, lethargy and collapse are mentioned.

As urological diseases, for example, cystitis and urolithiasis are mentioned.

As hepatic, biliary tract, and pancreatic diseases, for example, biliary sludge and chronic renal failure are mentioned.

As cardiovascular diseases, for example, valvular disease and cardiomyopathy are mentioned.

As neurological diseases, for example, epilepsy and convulsive seizures are mentioned.

As respiratory diseases, for example, there are mentioned coughing, rhinitis, tracheal collapse, and bronchial constriction.

As dental and oral diseases, for example, periodontal disease and stomatitis are mentioned.

As endocrine diseases, for example, hypothyroidism and diabetes are mentioned.

As genital system diseases, for example, mammary gland tumors and balanitis are mentioned.

As blood and hematopoietic diseases, for example, tumors of lymphoid tissue and thrombocytopenia are mentioned.

### [Output]

When the determining means of the present invention receives the occupancy rate data and diversity data of the intestinal flora of an animal as input information, the determining means makes, by the above learned model, a prediction determination of whether the animal will be afflicted with a disease within a predetermined period of time, preferably within 3 years, more preferably within 2 years, and still more preferably within 1 year from a time of acquisition of the occupancy rate data and diversity data of the intestinal flora.

The format of output is not particularly limited. The prediction determination can be output, for example, by giving an indication such as "likely to be afflicted with a disease within 1 year from now" or "less likely to be afflicted with a disease within 1 year from now" on a personal computer's screen.

When a learned model specified for each disease is generated, a prediction determination is made as to whether or not the animal will be afflicted with the disease within a predetermined period of time after receiving the occupancy rate data and diversity data of the intestinal flora. When a learned model is generated including a plurality of diseases, a prediction determination is made as to whether or not the animal will be afflicted with a specific disease among diseases included in teacher data within a predetermined period of time after receiving the occupancy rate data and diversity data of the intestinal flora.

The disease predicting system of the present invention may separately include output means of receiving a determination result from the determining means and outputting the determination result.

The disease predicting system of the present invention may further include deciding means of modifying a prediction result output from the determining means on the basis of a genetic test result of the animal input separately. The genetic test result is, for example, a result of detection of a genetic mutation or an SNP that is likely to cause disease. When the genetic test result is input to the disease predicting system through the input means, the disease predicting system can output a final prediction result by combining the genetic test result with the prediction result output from the determining means in the deciding means. Combining with the genetic test result enables highly accurate disease prediction.

The disease predicting system of the present invention may further include suggesting means of suggesting a life improvement method according to the disease prediction result. For example, the suggesting means can receive a prediction result output from the determining means and, according to the prediction result, can suggest or recommend disease-preventing meals, supplements containing bacteria that reduce susceptibility to disease, low-salt and low-calorie meals, low-carbohydrate meals, diet menus, and the like, for each predicted disease. The suggesting means may include a learned model.

Additionally, beverages, meals, and supplements for preventing a disease can also be produced or customized according to prediction results output by the disease predicting system or disease predicting method of the present invention. Services related to disease prevention can take a form of prediction, provision of prediction results, production or customization of beverages, meals, and supplements according to the prediction results, and suggestions and recommendations for the beverages, meals, and supplements by the disease predicting system or disease predicting method of the present invention. In addition, it is also possible to further implement the disease predicting method of the present invention after providing such services and present whether or not a tendency toward affliction to the disease has improved. The above beverages, meals, and supplements include dietary beverages, diet foods, dietary supplement additives, and the like.

In this way, by suggesting, producing, and customizing meals and foods according to the prediction results, it is expected to delay an onset of the disease and improve or alleviate disease conditions.

### [Insurance Premium Calculating System]

An insurance premium calculating system of the present invention inputs occupancy rate data and diversity data of intestinal flora of an animal covered by insurance to the above-described disease predicting system and determines an insurance premium for the animal according to the output prediction of disease affliction. In addition to the disease affliction prediction, information such as a facial image, species, breed, age, gender, weight, and medical history of the animal may be used to determine the insurance premium.

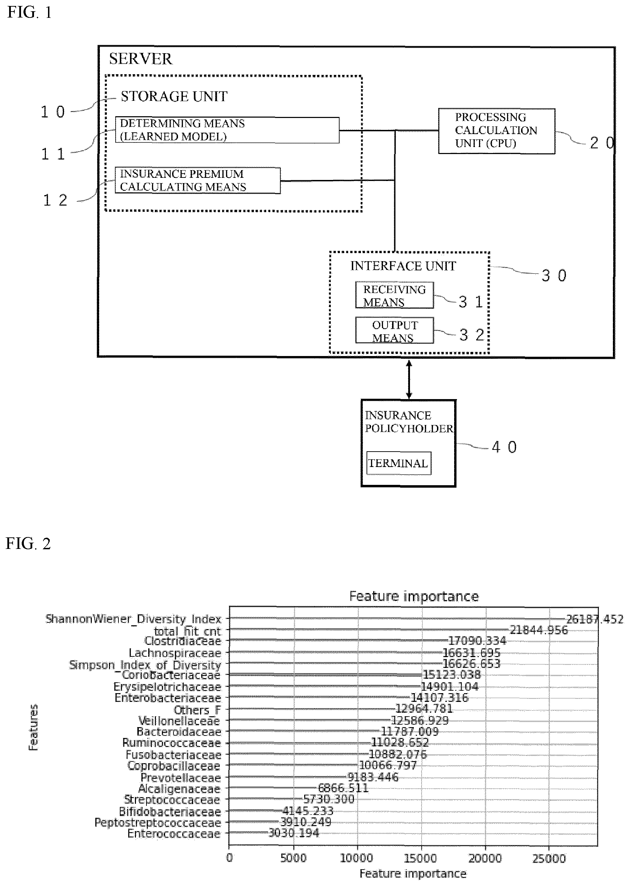

Hereinafter, an embodiment of the insurance premium calculating system of the present invention is described with reference to FIG. 1.

In FIG. 1, a terminal 40 is a terminal used by an insurance policyholder (user). As the terminal 40, for example, a personal computer, a tablet terminal, and the like are mentioned. The terminal 40 is configured by including a processing unit such as CPU, a storage unit such as a hard disk, ROM, or RAM, a display unit such as a liquid crystal panel, an input unit such as a mouse, keyboard, or touch panel, and a communication unit such as a network adapter.

The insurance policyholder accesses a server from the terminal 40, and inputs and transmits information such as occupancy rate data and diversity data of intestinal flora of an animal covered by insurance, a facial image (photograph), the animal's species and breed, and its age, weight, and medical history at the time of photography.

Additionally, the insurance policyholder can receive a disease prediction result and an insurance premium calculation result in the server by accessing the server through the terminal 40.

In addition, the insurance policyholder receives a delivery of a fecal sample collection kit for examining the intestinal flora of the pet to be insured, and sends a fecal sample to an intestinal flora measurement company (not shown). The company performs measurement of the intestinal flora of the pet and acquires occupancy rate data and diversity data of the intestinal flora. Then, the company may input and transmit the occupancy rate data and diversity data of the intestinal flora of the pet to receiving means 31 of the server directly via its own terminal, or the company may send the occupancy rate data and diversity data of the intestinal flora of the pet to the insurance policyholder otherwise such as by mail or e-mail, and the insurance policyholder may input and transmit the occupancy rate data and diversity data of the intestinal flora to the receiving means 31 through the terminal 40.

In the present embodiment, the server is configured by a computer, but may be any device as long as it has functions according to the present invention.

The storage unit 10 is composed of, for example, ROM, RAM, a hard disk, or the like. The storage unit 10 stores an information processing program for operating each unit of the server, and particularly stores determining means (learned model) 11 and, if necessary, insurance premium calculating means 12. The insurance premium calculating means 12 may be omitted when the system is configured as a disease predicting system that simply outputs predictions of disease afflictions without the purpose of calculating insurance premiums.

As described above, the determining means (learned model) 11 takes as an input the occupancy rate data and diversity data of the intestinal flora of the animal covered by insurance input by the insurance policyholder or the company that performed the measurement of the intestinal flora, and outputs a prediction of whether or not the animal will be afflicted with a specific disease within a predetermined period of time (for example, within half a year) from the time of acquisition of the occupancy rate data and diversity data of the intestinal flora or the time of input of the occupancy rate data and diversity data of the intestinal flora. The determining means (learned model) 11 in the present embodiment is configured by including, for example, XGBoost, CatBoost, LightGBM, a deep neural network, or a convolutional neural network.

The insurance premium calculating means 12 is software that calculates an insurance premium for an animal from the prediction of disease affliction output by the above determining means 11 and the information such as the animal's species and breed, and its age, weight, and medical history at the time of acquisition of the occupancy rate data and diversity data of the intestinal flora input by the insurance policyholder. For example, the software is software that grades insurance premiums according to the animal's species and breed, and its age, weight, and medical history at the time of acquisition of the occupancy rate data and diversity data of the intestinal flora, and the like, and finally modifies the grade by taking into account the prediction of disease affliction output by the above determining means 11 to calculate a final insurance premium.

The insurance premium calculating means 12 and the determining means (learned model 11 may be a single piece of software.

A processing calculation unit 20 calculates a prediction of disease affliction and an insurance premium using the determining means (learned model) 11 and the insurance premium calculating means 12 stored in the storage unit.

An interface unit (communication unit) 30 includes the receiving means 31 and output means 32, receives the occupancy rate data and diversity data of the intestinal flora of the animal and the other information from the terminal of the insurance policyholder, and outputs a prediction of disease affliction and a result of insurance premium calculation to the terminal of the insurance policyholder.

With the insurance premium calculating system of the present embodiment, insurance policyholders can send samples, such as fecal samples of animals, in addition to application for pet insurance to obtain pet insurance premiums and prediction results of disease affliction in the future at the same time that a card (pet health insurance card) or the like indicating pet insurance coverage is created.

### [Number of Bacterial Species]

The disease predicting system of the present invention may use data on the number of bacterial species of each family in addition to occupancy rate data and diversity data of intestinal flora, as teacher data and input data.

### [Preferable Families]

As described above, the occupancy rate in occupancy rate data of the present invention may be an occupancy rate at any level of phylum, class, order, family, genus, or species of bacteria, but an occupancy rate of each bacterial family is preferred. Then, as families, preferred are one or more families selected from the group consisting of Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, and Veillonellaceae.

Additionally, in the present invention, as a family, Streptococcaceae is preferable in addition to the above. In other words, preferred are one or more families selected from the group consisting of Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, Veillonellaceae, and Streptococcaceae.

Additionally, in the present invention, furthermore in addition to the above, one or more families selected from the group consisting of Campylobacteraceae, Desulfovibrionaceae, Flavobacteriaceae, Helicobacteraceae, Odoribacteraceae, Paraprevotellaceae, Peptococcaceae, Porphyromonadaceae, and Succinivibrionaceae are preferable. In other words, preferred are one or more families selected from the group consisting of Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, Veillonellaceae, Campylobacteraceae, Desulfovibrionaceae, Flavobacteriaceae, Helicobacteraceae, Odoribacteraceae, Paraprevotellaceae, Peptococcaceae, Porphyromonadaceae, and Succinivibrionaceae.

### [Alcaligenaceae]

Alcaligenaceae is a family belonging to the order Burkholderiales, and includes the genus Alcaligenes.

### [Bacteroidaceae]

Bacteroidaceae is a family belonging to the order Bacteroidales of the class Bacteroidia of the phylum Bacteroidetes, and includes the genus Bacteroides.

### [Bifidobacteriaceae]

Bifidobacteriaceae is a family included in the order Bifidobacteriales, and includes the genus Bifidobacterium.

### [Clostridiaceae]

Clostridiaceae is a family of the order Clostridiales, and includes the genus Clostridium.

### [Coprobacillaceae]

Coprobacillaceae is a family belonging to the order Erysipelotrichales, and includes the genus Coprobacillus.

### [Coriobacteriaceae]

Coriobacteriaceae is a family belonging to the order Coriobacteriales, and includes, as genera, Collinsella, Coriobacterium, Atopobium, and Olsenella.

### [Enterobacteriaceae]

Enterobacteriaceae is a family belonging to the order Enterobacterales of the class Gammaproteobacteria of the phylum Proteobacteria. Enterobacteriaceae includes, as genera, for example, the genera Enterobacter, Escherichia, Klebsiella, Salmonella, Serratia, Yersinia, Arsenophonus, Biostraticola, Candidatus Blochmannia, Brenneria, Buchnera, Budvicia, Buttiauxella, Cedecea, Citrobacter, Cosenzaea, Cronobacter, Cickeya, Dickeya, Edwardsiella, Erwinia, and the like.

### [Enterococcaceae]

Enterococcaceae is a family of gram-positive eubacteria belonging to the order Lactobacillus. Representative genera include Enterococcus, Melissococcus, Pilibacter, Tetragenococcus, and Vagococcus.

### [Erysipelotrichaceae]

Erysipelotrichaceae is a family belonging to the order Erysipelotrichales, and includes, as genera, Allobaculum, Breznakia, Bulleidia, Catenibacterium, Catenisphaera, Coprobacillus, Dielma, Dubosiella, Eggerthia, Erysipelothrix, Faecalibaculum, Holdemania, Ileibacterium, Kandleria, Longicatena, Solobacterium, and Turicibacter.

### [Fusobacteriaceae]

Fusobacteriaceae is a family belonging to the order Fusobacteriales, and includes the genus Fusobacterium.

### [Lachnospiraceae]

Lachnospiraceae is a family included in the order Clostridiales of the class Clostridia, and includes the genus Lachnospira.

### [Peptostreptococcaceae]

Peptostreptococcaceae is a family included in the order Clostridiales of the class Clostridia, and includes the genus Peptostreptococcus.

### [Prevotellaceae]

Prevotellaceae is a family belonging to the order Bacteroidales, and includes the genus Prevotella.

### [Ruminococcaceae]

Ruminococcaceae is a family of bacteria belonging to the order Clostridiales of the class Clostridia. In addition to the genus Ruminococcus, genera include Acetanaerobacterium, Acutalibacter, Anaerobacterium, Anaerofilum, Anaerotruncus, Dysosmobacter, Ercella, Ethanoligenens, Faecalibacterium, Fastidiosipila, Hydrogenoanaerobacterium, Oscillibacter, Oscillospira, Papillibacter, Pseudobacteroides, Sporobacter, Subdoligranulum, and Youngiibacter.

### [Veillonellaceae]

Veillonellaceae is a family belonging to the order Clostridiales, and includes, as genera, Acetonema, Acidaminococcus, Allisonella, Anaeroarcus, Anaeroglobus, Anaeromusa, Anaerosinus, Anaerospora, Anaerovibrio, Centipeda, Dendrosporobacter, Desulfosporomusa, Dialister, Megamonas, Megasphaera, Mitsuokella, Pectinatus, Pelosinus, Phascolarctobacterium, Propionispira, Propionispora, Psychrosinus, Quinella, Schwartzia, Selenomonas, Sporomusa, Sporotalea, Succiniclasticum, Succinispira, Thermosinus, Veillonella, and Zymophilus.

### [Streptococcaceae]

Streptococcaceae is placed in the order Lactobacillales, and includes the genera Lactococcus, Lactovum, and Streptococcus.

### [Campylobacteraceae]

Campylobacteraceae is a family belonging to the order Campylobacterales of the class Epsilonproteobacteria, and includes the genus Campylobacter.

### [Desulfovibrionaceae]

Desulfovibrionaceae is a family belonging to the order Desulfovibrionales of the class deltaproteobacteria, and includes the genus Desulfovibrio.

### [Flavobacteriaceae]

Flavobacteriaceae is a family belonging to the order Flavobacteriales of the class Flavobacteria, and includes the genus Flavobacterium.

### [Helicobacteraceae]

Helicobacteraceae is a family belonging to the order Campylobacterales of the class Epsilonproteobacteria, and includes the genus Helicobacter.

### [Odoribacteraceae]

Odoribacteraceae is a family belonging to the order Bacteroidales of the class Bacteroidia, and includes the genus Odoribacter.

### [Paraprevotellaceae]

Paraprevotellaceae is a family belonging to the order Bacteroidales, and includes the genus Paraprevotella. Additionally, there is also a classification method that includes Paraprevotella in Prevotellaceae without regarding Paraprevollaceae as a separate family.

### [Peptococcaceae]

Peptococcaceae is a family belonging to the order Clostridiales, and includes the genus Peptococcus.

### [Porphyromonadaceae]

Porphynomonadaceae is a family belonging to the order Bacteroidales of the class Bacteroidia, and includes the genus Porphynomonas.

### [Succinivibrionaceae]

Succinivibrionaceae is a family belonging to the order Aeromonadales of the class Gammaproteobacteria, and includes the genus Aeromonas and the genus Anaerobiospirillum.

### EXAMPLES

Examples of the present invention are shown hereinbelow. The present invention is not limited to the following Examples.

### [Example 1]

### (DNA Extraction from Fecal Samples)

DNA was extracted by collecting a fecal sample from each dog in the following manner.

Dog owners collected dog fecal samples using a fecal collection kit. The fecal samples were received and suspended in water.

Next, 200 µL of the fecal suspension and 810 µL of Lysis buffer (containing 224 µg/mL of Protenase K) were added to a bead tube, and bead crushing (6,000 rpm, 20-second crushing, a 30-second interval, and 20-second crushing) was performed with a bead homogenizer. After that, the samples were left on a heat block at 70°C for 10 minutes to be treated with Protenase K, and then left on a heat block at 95°C for 5 minutes to inactivate the Protenase K. The lysed samples were subjected to automated DNA extraction using chemagic 360 (PerkinElmer) with a chemagic kit stool protocol to obtain 100 µL of DNA extract.

### (Meta 16S RNA Gene Sequence Analysis)

Meta 16S sequence analysis was performed by modifying illumina 16S Metagenomic Sequencing Library Preparation (version 15044223 B). First, a 460 bp region of a 16S rRNA gene containing variable regions V3V4 was amplified by PCR using universal primers (Illumina_16S_341F and Illumina_16S_805RPCR). A PCR reaction solution was prepared by mixing 10 µL of the DNA extract, 0.05 µL of each primer (100 µM), 12.5 µL of 2x KAPA HiFi Hot-Start Ready Mix (F. Hoffmann-LaRoche, Switzerland), and 2.4 µL of PCR grade water. The PCR involved heat denaturation at 95°C for 3 minutes, followed by 30 cycles of 95°C for 30 seconds, 55°C for 30 seconds, and 72°C for 30 seconds, and finally an elongation reaction at 72°C for 5 minutes. Each amplified product was purified using magnetic beads and eluted with 50 µL of Buffer EB (QIAGEN, Germany). The amplified product after purification was subjected to PCR using Nextera XT Index Kit v2 (Illumina, CA, US) to add an index. A PCR reaction solution was prepared by mixing 2.5 µL of the amplified product, 2.5 µL of each primer, 12.5 L of 2x KAPA HiFi Hot-Start Ready Mix, and 5 µL of PCR grade water. The PCR involved heat denaturation at 95°C for 3 minutes, followed by 12 cycles of 95°C for 30 seconds, 55°C for 30 seconds, and 72°C for 30 seconds, and finally an elongation reaction at 72°C for 5 minutes. The indexed amplified products were purified using magnetic beads and eluted with 80105 µL of Buffer EB. The concentration of each amplified product was measured with a NanoPhotometer (Implen, CA, US) and prepared at 1.4 nM, followed by mixing in equal amounts to generate a library for sequencing. DNA concentration of the sequencing library and sizes of the amplified products were confirmed by electrophoresis and analyzed by MiSeq. A MiSeq Reagent Kit V3 was used for the analysis, and 2 × 300 bp paired-end sequencing was performed. The resulting sequences were analyzed by MiSeq Reporter to obtain bacterial composition data.

The sequences of the universal primers used above are as follows. The universal primers can be purchased commercially.
Illumina_16S_341F 5'-TCGTCGGCAGCGTCAGATGTGTATAAGAGACAGCCTACGGGNGGCWGCAG-3'
Ilumina_16S_805R 5'-GTCTCGTGGGCTCGGAGATGTGTATAAGAGACAGGACTACHVGGGTATCTAATCC-3'

According to the above method, composition data of intestinal flora of all dog breeds of 165, 040 individuals aged 0 and over were obtained, and occupancy rates and diversity indices were measured for each family.

The occupancy rate of each family was calculated as a hit rate of bacteria classified into the family.

Shannon-Winner's diversity index and Simpson's diversity index were calculated using QIIME 2.

### (Investigation of Presence or Absence of Disease Affliction)

For all the above dog breeds, it was investigated whether they had been afflicted with a disease within 180 days after the fecal sample collection for each. The presence or absence of disease affliction was investigated by using insurance claim records of pet insurance to determine whether or not any insurance claim had been filed within 180 days after a date of the fecal collection for each individual for which the fecal collection had been made. Additionally, regardless of disease affliction, investigation was conducted on policies for which no insurance claim had been filed within 90 days before the date of the fecal collection. The absence of any insurance claim within 90 days before the date of the fecal collection gives a presumption that there was no disease affliction before the fecal collection.

### (Generation of Learned Model)

Among the occupancy rates of the intestinal flora of all the dog breeds obtained above, the respective occupancy rates of the families Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, Veillonellaceae, and Streptococcaceae were labeled as "0", "1", "2", ..., "19" according to a magnitude of value, and were used as features related to occupancy rate data. Additionally, the ShannonWiener's diversity index and the Simpson's diversity index were also labeled to be features related to diversity data. The reason why the above families were chosen is that among approximately 300 families known to be present in the intestinal flora of dogs, families with a hit rate of 0 in the canine intestinal flora of 80% or more of all the dogs (165,040 dogs) were eliminated, and the remaining families were chosen.

On the other hand, the presence or absence of disease affliction was replaced with a dummy variable to be a feature.

Machine learning was performed using the above occupancy rate data and diversity data and the presence or absence of disease affliction as teacher data to generate a learned model.

Learning was performed using LightGBM, a machine learning framework for gradient boosting based on a decision tree algorithm.

The number of teacher data samples for each disease was as shown in Tables 1 and 2 below.

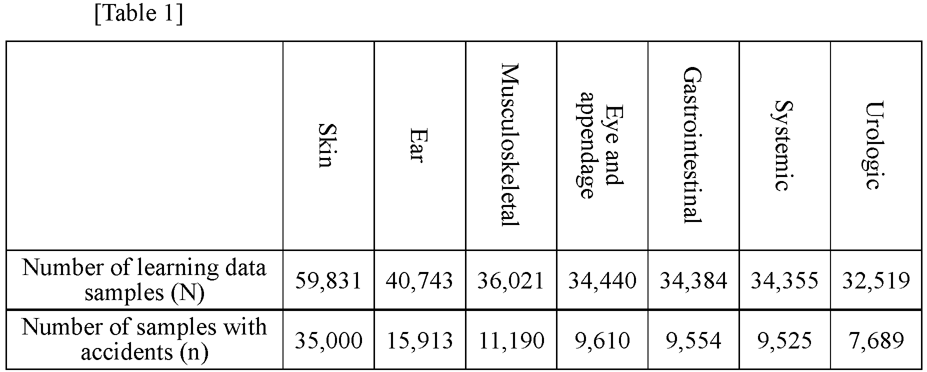

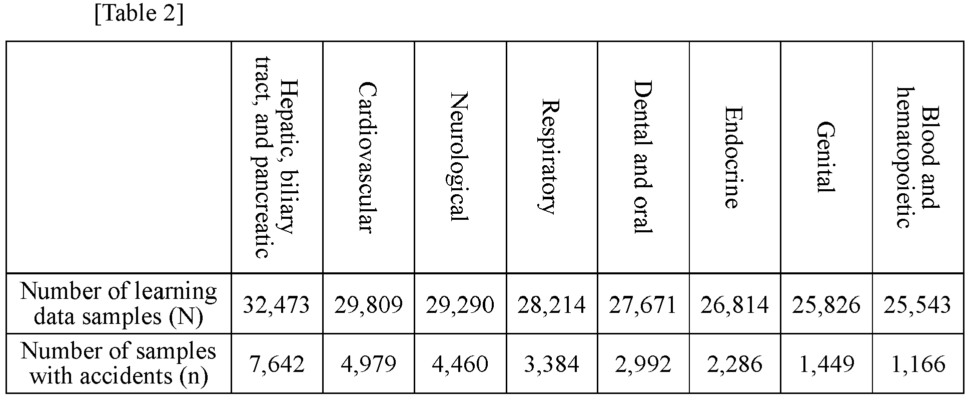

### (Prediction of Disease Affliction Using Learned Model)

Disease affliction was predicted for dogs using the learned model generated above.

Measurement of intestinal flora from fecal samples of 300 to 700 dogs different from the dogs used for the above machine learning was conducted to obtain occupancy rate data and diversity data. These occupancy rate data and diversity data were input to the learned model to predict disease affliction.

Whether the prediction results were correct or incorrect depended on the presence or absence of disease affliction within 180 days after collection of the fecal samples. Predictive performance was evaluated primarily by an Area Under the ROC Curve (AUC). Predictive performance was evaluated as "A" when the AUC in the data used to confirm predictive performance was 85% or more, "B" when it was from 75% to less than 85%, and "C" when it was less than 75%. Table 3 shows the results.

**[Table 3]**

| Disease name | Predictive performance | Disease name | Predictive performance |
|---|---|---|---|
| Skin | A | Cardiovascular | A |
| Ear | A | Neurological | A |
| Musculoskeletal | B | Respiratory | A |
| Eye and appendage | B | Dental and oral | B |
| Gastrointestinal | B | Endocrine | A |
| Systemic | B | Genital | B |
| Urologic | A | Blood and hematopoietic | A |
| Hepatic, biliary tract, and pancreatic | A | * Predictive performance was determined by AUC value. A: 85% or more; B: from 75% to less than 85%; and C: less than 75% | |

As is clear from Table 3, the disease predicting system including the learned model of the present invention showed high predictive performance for various diseases.

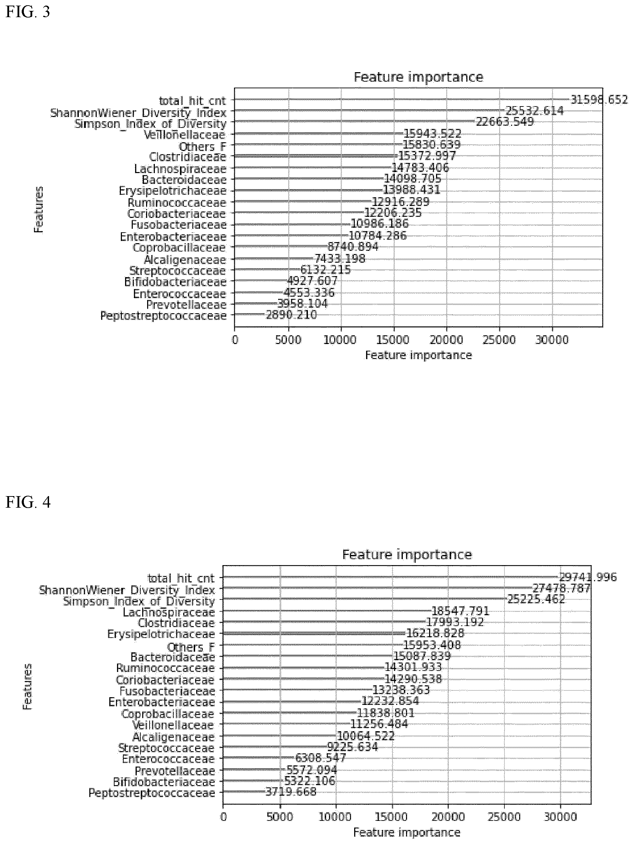

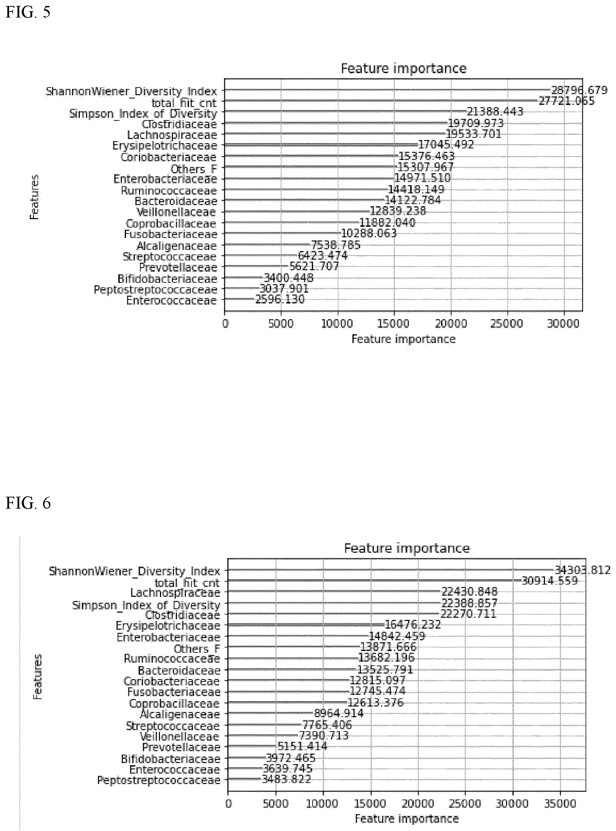

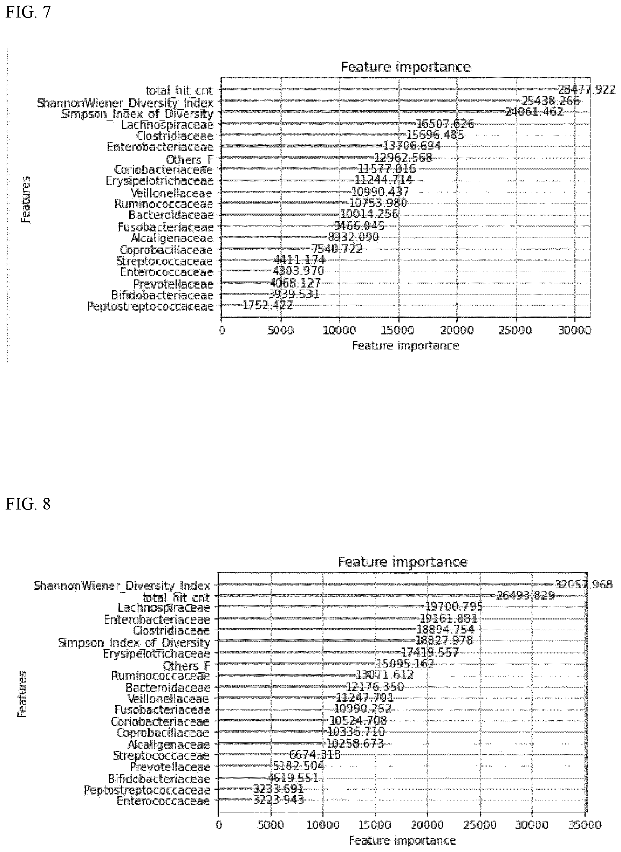

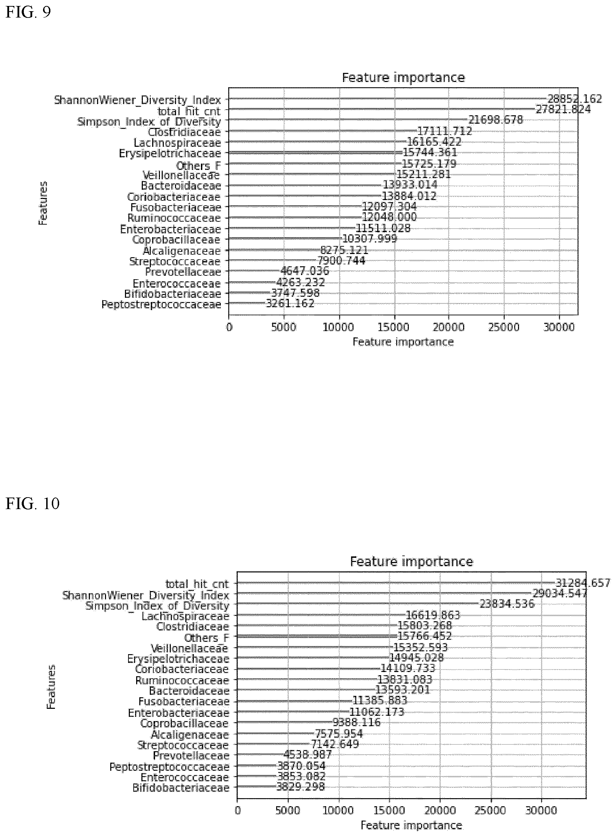

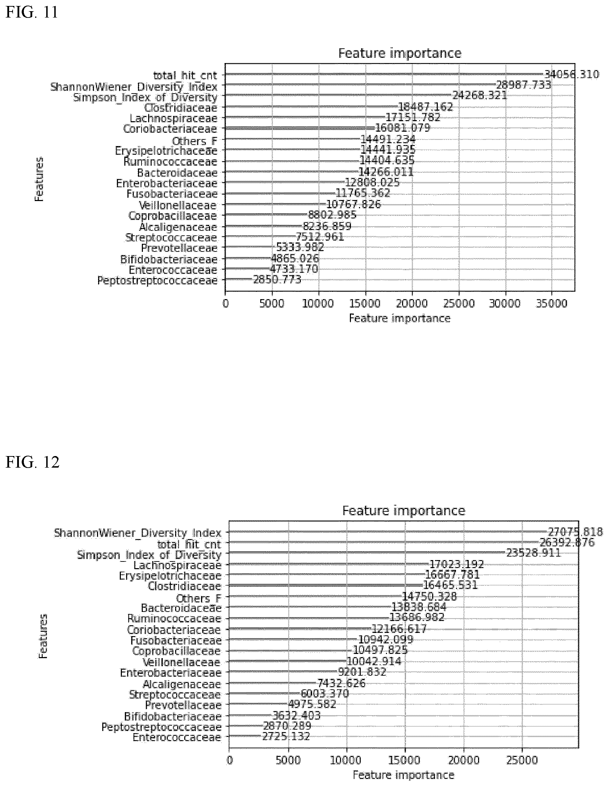

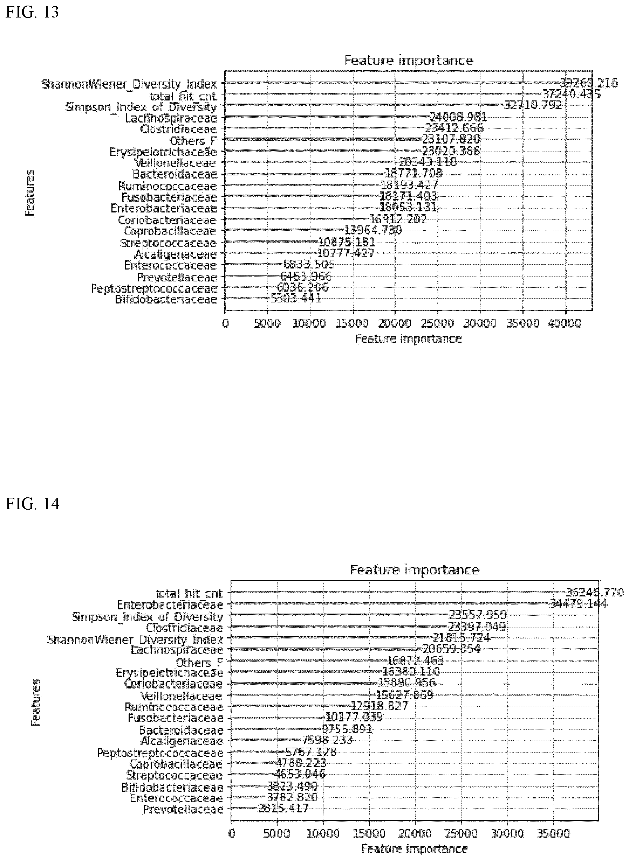

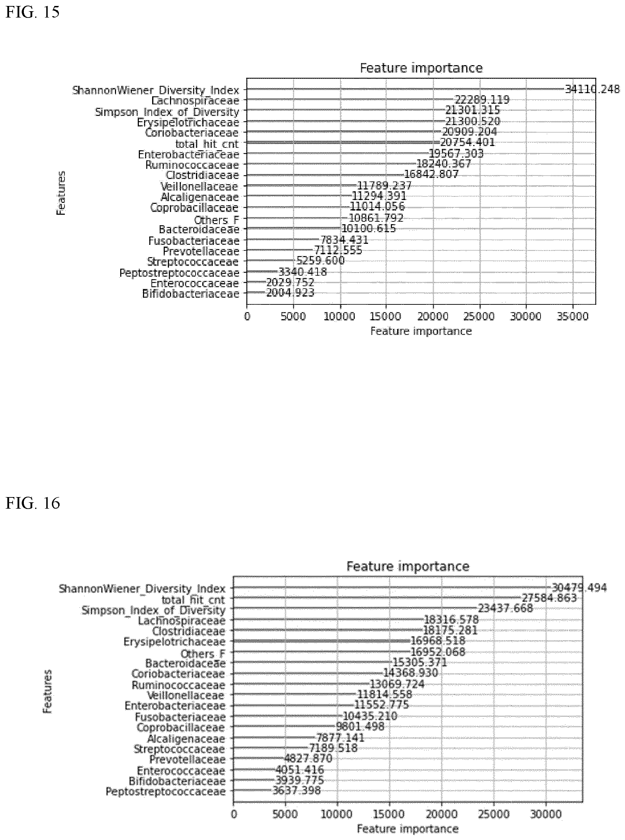

In addition, FIGS. 2 to 16, respectively, show features that were important for prediction of each disease. FIGS. 2 to 16 are graphs in which the features used for the prediction are arranged in order of importance and visualized. Values listed in the graphs represent how much, in a finally completed model, the features were able to improve evaluation criteria in the prediction model on average, and the values themselves are not so important and can be understood to have meanings as relative values. FIGS. 2 to 16 suggest that not all of the above families are essential for prediction, and disease prediction can be made even if some of the above families are used as features. Note that "Others F" refers to families other than Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, Veillonellaceae, and Streptococcaceae. The "Other_F" is an adjustment term when the sum of hit rates is not equal to 1.

### [Example 2]

### (DNA Extraction from Fecal Samples)

In the same manner as in the dog example above, fecal samples were collected from cats and DNA was extracted.

### (Meta 16S RNA Gene Sequence Analysis)

Meta 16S sequence analysis was performed on the fecal samples of the cats in the same manner as in the above dog example to obtain bacterial composition data of intestinal flora.

In the same manner as in the above dog example, the composition data of the intestinal flora were obtained for all cat breeds of 31, 040 individuals aged 0 and over, and occupancy rates and diversity indices were measured for each family.

The occupancy rate of each family was calculated as a hit rate of bacteria classified into the family.

Shannon-Winner's diversity index and Simpson's diversity index were calculated using QIIME 2.

### (Investigation of Presence or Absence of Disease Affliction)

For the above cats, it was investigated whether they had been afflicted with a disease within 180 days after the fecal sample collection for each. The presence or absence of disease affliction was investigated by using insurance claim records of pet insurance to determine whether or not any insurance claim had been filed within 180 days after a date of the fecal collection for each individual for which the fecal collection had been made. Additionally, regardless of disease affliction, the investigation was conducted on policies for which no insurance claim had been filed within 90 days before the date of the fecal collection. The absence of any insurance claim within 90 days before the date of the fecal collection gives a presumption that there was no disease affliction before the fecal collection.

### (Generation of Learned Model)

Among the occupancy rates of the intestinal flora of the cats obtained above, the respective occupancy rates of the families Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, Veillonellaceae, Campylobacteraceae, Desulfovibrionaceae, Flavobacteriaceae, Helicobacteraceae, Odoribacteraceae, Paraprevotellaceae, Peptococcaceae, Porphyromonadaceae, and Succinivibrionaceae were labeled as "0", "1", "2", ..., "19" according to the magnitude of value, and were used as features related to occupancy rate data. Additionally, the ShannonWiener's diversity index and the Simpson's diversity index were also labeled to be features related to diversity data. The reason why the above families were chosen is that among approximately 300 families known to be present in the intestinal flora of cats, families with a hit rate of 0 in the feline intestinal flora of 80% or more of all the cats (31,040 cats) were eliminated, and the remaining families were chosen.

On the other hand, the presence or absence of disease affliction was replaced with a dummy variable to be a feature.

Machine learning was performed using the above occupancy rate data and diversity data and the presence or absence of disease affliction as teacher data to generate a learned model.

The machine learning is similar to the above dog example.

The number of teacher data samples for each disease was as shown in Tables 4 and 5 below.

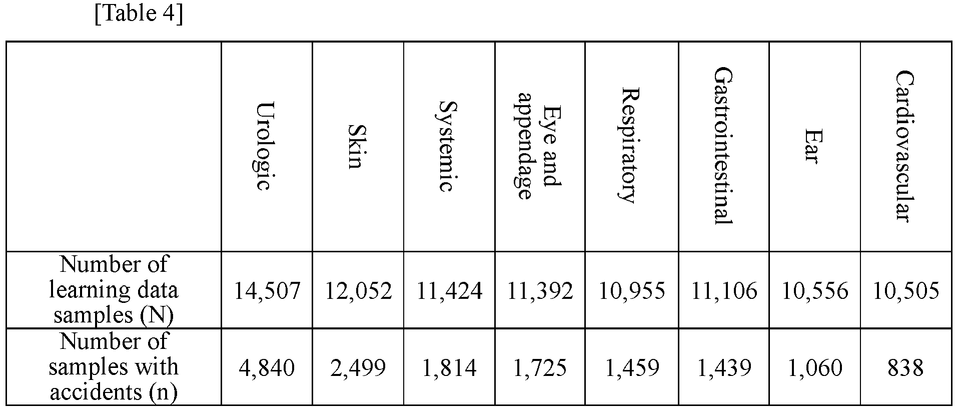

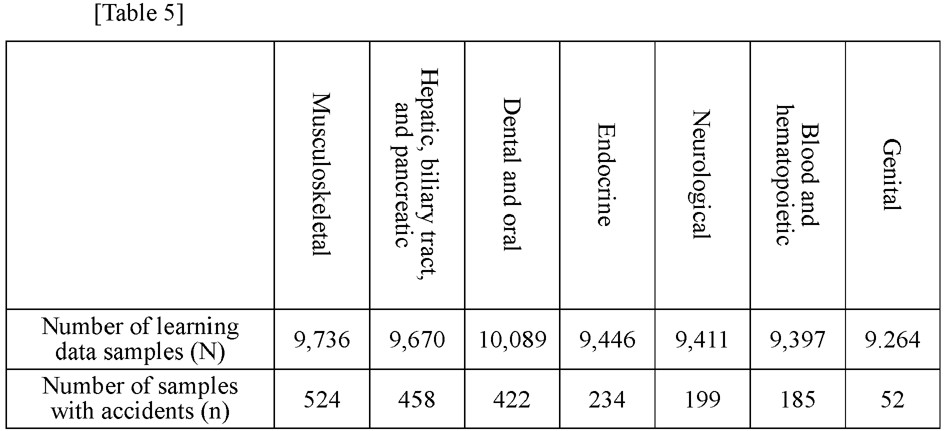

### (Prediction of Disease Affliction Using Learned Model)

Disease affliction was predicted for cats using the learned model generated above.

Measurement of intestinal flora from fecal samples of 150 to 3350 cats different from the cats used in the above machine learning was conducted to obtain occupancy rate data and diversity data. These occupancy rate data and diversity data were input to the learned model to predict disease affliction.

Whether the prediction results were correct or incorrect depended on the presence or absence of disease affliction within 180 days after collection of the fecal samples. Predictive performance was evaluated primarily by an Area Under the ROC Curve (AUC). Predictive performance was evaluated as "A" when the AUC in the data used to confirm predictive performance was 85% or more, "B" when it was from 75% to less than 85%, and "C" when it was less than 75%. Table 6 shows the results.

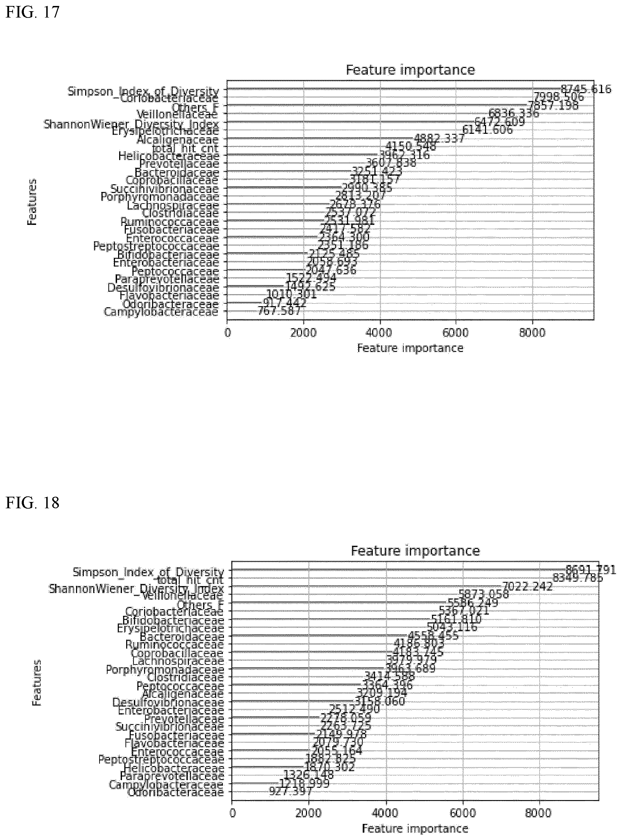

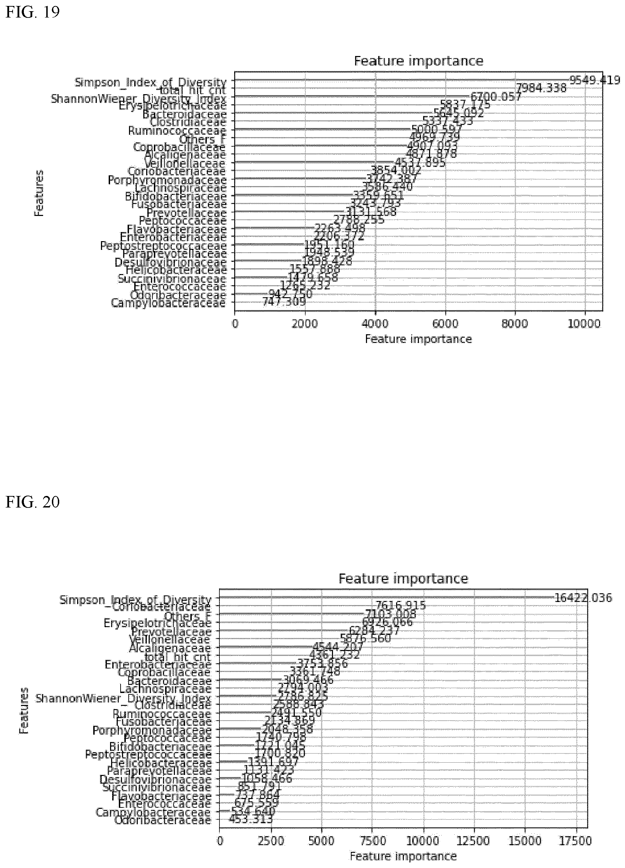

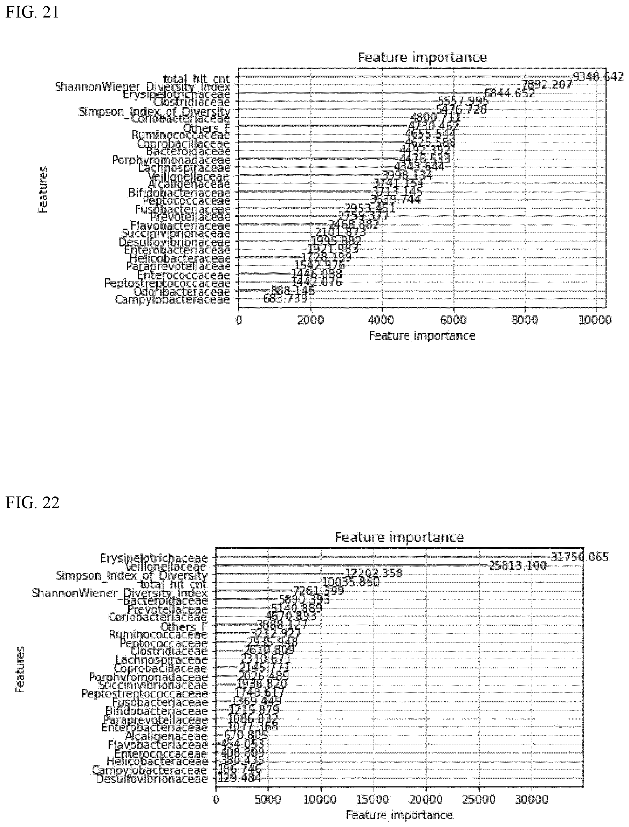

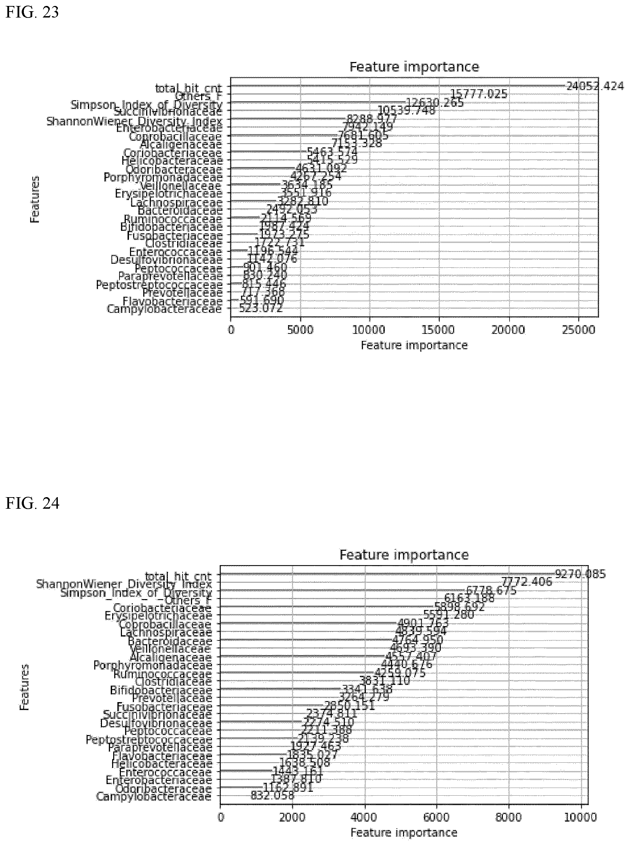

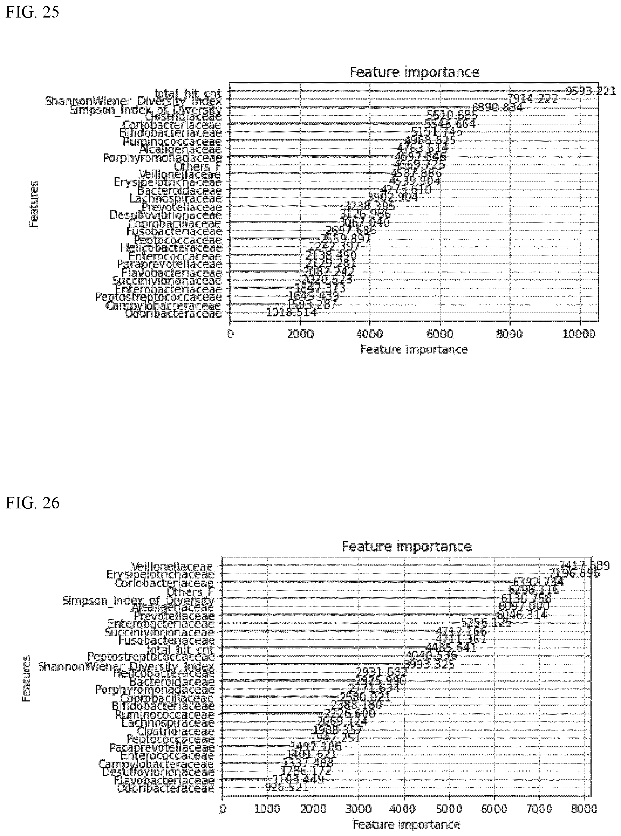

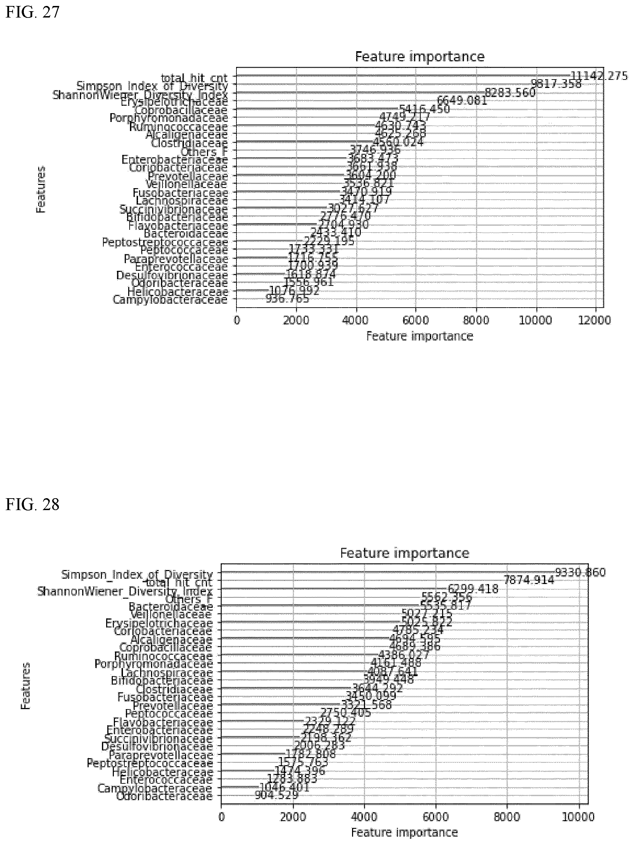

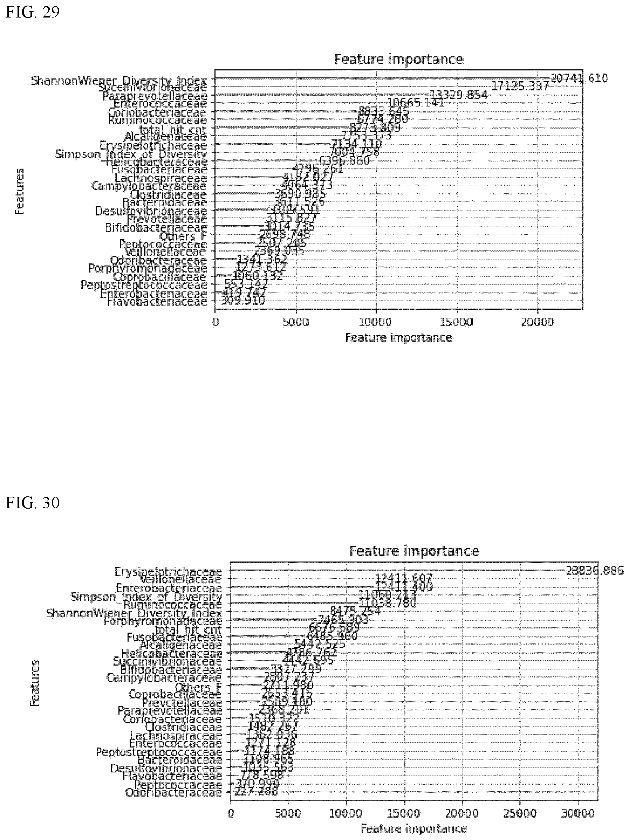

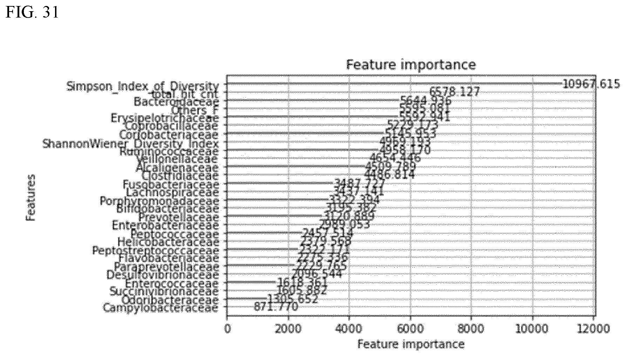

In addition, FIGS. 17 to 31, respectively, show features that were important for prediction of each disease. In the same manner as FIGS. 2 to 16, FIGS. 17 to 31 are graphs in which the features used for the prediction are arranged in order of importance and visualized.

**[Table 6]**

| Disease name | Predictive performance | Disease name | Predictive performance |
|---|---|---|---|
| Urological | A | Musculoskeletal | B |
| Skin | B | Hepatic, biliary tract, and pancreatic | A |
| Systemic | B | Dental and oral | B |
| Eye and appendage | B | Endocrine | A |
| Respiratory | B | Neurological | A |
| Gastrointestinal | A | Blood and hematopoietic | A |
| Ear | B | Genital | B |
| Cardiovascular | A | | |

As is clear from Table 6, the disease predicting system including the learned model of the present invention showed high predictive performance for various diseases.

### [Example 3]

For each feature of occupancy rate data and diversity data of dogs obtained in the same manner as above, machine learning was performed using features listed in Table 7 below to generate a learned model. In addition, target diseases were skin diseases.

**[Table 7]**

| | Skin | Skin | Skin | Skin |
|---|---|---|---|---|
| Number of learning data samples (N) | 59,831 | 59,831 | 59,831 | 59,831 |
| Number of samples with accidents (n) | 35,000 | 35,000 | 35,000 | 35,000 |
| Ratio (n/N) | 58% | 58% | 58% | 58% |
| AUC | 90% | 89% | 89% | 88% |
| Important feature_1 | ShannonWiener_ Diversity_Index | Simpson_Index_ of_Diversity | ShannonWiener_ Diversity_Index | ShannonWiener_ Diversity_Index |
| Important feature_2 | total_hit_cnt | total_hit_cnt | total_hit_cnt | Simpson_Index_ of Diversity |
| Important feature 3 | Simpson_Index_ of Diversity | ShannonWiener_ Diversity_Index | Simpson_Index_ of Diversity | total_hit_cnt |
| Important feature 4 | Lachnospiraceae | Others_F | Lachnospiraceae | Clostridiaceae |
| Important feature_5 | Clostridiaceae | Veillonellaceae | Clostridiaceae | Others_F |
| Important feature_6 | Others_F | Erysipelotrichaceae | Others_F | Lachnospiraceae |
| Important feature_7 | Erysipelotrichaceae | Fusobacteriaceae | Erysipelotrichaceae | - |
| Important feature_8 | Veillonellaceae | Clostridiaceae | Veillonellaceae | - |
| Important feature 9 | Bacteroidaceae | Bacteroidaceae | Bacteroidaceae | - |
| Important feature 10 | Ruminococcaceae | Enterobacteriaceae | Ruminococcaceae | - |

As is clear from the AUC in Table 7 above, even when 10 types, 7 types, and 3 types were used as bacterial families, highly accurate disease prediction was still possible.

## Claims

1. A disease predicting system comprising: receiving means of receiving occupancy rate data and diversity data of intestinal flora of an animal other than humans; and determining means of predicting and determining, using a learned model, whether the animal will be afflicted with a disease from the occupancy rate data and diversity data of the intestinal flora of the animal input to the receiving means.

2. The disease predicting system according to claim 1, wherein the learned model performs learning using as teacher data the occupancy rate data and diversity data of the intestinal flora of the animal other than humans and a presence or absence of disease affliction within a predetermined period of time from a time of acquisition of the occupancy rate data and diversity data of the intestinal flora of the animal, and wherein an input is occupancy rate data and diversity data of intestinal flora of an animal and an output is a prediction determination of whether the animal will be afflicted with a disease.

3. The disease predicting system according to claim 2, wherein the occupancy rate data includes an occupancy rate of each family or a label set based on the occupancy rate of each family.

4. The disease predicting system according to claim 3, wherein the occupancy rate data includes an occupancy rate of one or more families selected from the group consisting of Alcaligenaceae, Bacteroidaceae, Bifidobacteriaceae, Clostridiaceae, Coprobacillaceae, Coriobacteriaceae, Enterobacteriaceae, Enterococcaceae, Erysipelotrichaceae, Fusobacteriaceae, Lachnospiraceae, Peptostreptococcaceae, Prevotellaceae, Ruminococcaceae, and Veillonellaceae or a label set based on the occupancy rate.

5. The disease predicting system according to claim 4, wherein the occupancy rate data further includes an occupancy rate of Streptococcaceae or a label set based on the occupancy rate.

6. The disease predicting system according to claim 4, wherein the occupancy rate data further includes an occupancy rate of one or more families selected from the group consisting of Campylobacteraceae, Desulfovibrionaceae, Flavobacteriaceae, Helicobacteraceae, Odoribacteraceae, Paraprevotellaceae, Peptococcaceae, Porphyromonadaceae, and Succinivibrionaceae or a label set based on the occupancy rate.

7. The disease predicting system according to any one of claims 1 to 6, wherein the diversity data includes a diversity index or a label set based on a basis of the diversity index.

8. An insurance premium calculating system for inputting occupancy rate data and diversity data of intestinal flora of an animal covered by insurance to the disease predicting system according to any one of claims 1 to 7 and determining an insurance premium for the animal according to the output prediction of disease affliction.

9. A learned model generating method for generating a learned model for predicting from occupancy rate data and diversity data of intestinal flora of an animal other than humans whether the animal will be afflicted with a disease within a predetermined period of time, the method comprising: inputting as teacher data occupancy rate data and diversity data of intestinal flora of an animal other than humans and a presence or absence of disease affliction within a predetermined period of time from a time of acquisition of the occupancy rate data and diversity data of the intestinal flora of the animal to a computer including artificial intelligence; and making artificial intelligence learn.

10. A disease predicting method comprising: a step of acquiring occupancy rate data and diversity data of intestinal flora of an animal other than humans; and a step of inputting the occupancy rate data and diversity data to a learned model and causing a computer to output, using the learned model, a prediction of whether the animal will be afflicted with a disease within a predetermined period of time from the input occupancy rate data and diversity data.

11. The disease predicting method according to claim 10, wherein the learned model performs learning using as teacher data the occupancy rate data and diversity data of the intestinal flora of the animal other than humans and a presence or absence of disease affliction within a predetermined period of time from a time of acquisition of the occupancy rate data and diversity data of the intestinal flora of the animal, and wherein an input is occupancy rate data and diversity data of intestinal flora of an animal and an output is a prediction determination of whether the animal will be afflicted with a disease.
